# EUROPEAN PATENT APPLICATION

(11) **EP 1 873 237 A1**
(43) Date of publication of application: **02.01.2008**
(21) Application number: 07252565.2
(22) Date of filing: 25.06.2007
(51) Int. Cl.: C12N 5/06, C12N 5/08

(54) **Growth of embryonic stem cells**

(30) Priority: 26.06.2006 US 816514 P
(71) Applicant: MILLIPORE CORPORATION, Billerica Massachusetts 01821 (US)
(72) Inventor: Sheridan, Steven D., Wakefield, MA 01880 (US); Gill, Sonia, Gloucester, MA 01930 (US)
(74) Representative: Kirkham, Nicholas Andrew

(57) **Abstract**

The invention provides systems, kits and methods relating to the growth of stem cells.

## Description

The invention relates generally to the field of cell biology. In certain specific embodiments the invention provides systems, kits and methods relating to the growth of stem cells.

### Background of the Invention

Embryonic stem cells (ESCs) may be derived from pre-embryonic, embryonic, or fetal tissue at any time after fertilization. The isolation and growth of various types of stem cells have been previously described, see, e.g., Robertson, 1997, Methods of Cell Biology 75:173; Thompson et al. 1995, Proc. Natl. Acad. Sci. USA 92:7844; Thompson et al. 1998, Science 282:114; U.S. Patent Nos. 5,166,065; 5,332,672; 5,405,772; 5,453,357; 5,639,618; 5,672,499; 5,843,780; 5,914,268; 5,922,597; 5,968,829; 6,040,180; 6,090,622; 6,833,269; 6,506,574; 6,458,589; 6,667,176; 7,041,438; International Patent Publication WO 99/20741.

When cultured under the appropriate conditions ESCs are capable of indefinite proliferation in vitro in an undifferentiated state, retain a normal karyotype, and retain the ability to differentiate to derivatives of all three embryonic germ layers: endoderm, mesoderm and ectoderm.

ESCs have potential wide ranging applications. They may be useful in the area of drug discovery, and basic scientific research to name but a few. Early discovery in the drug development process involves target identification and validation. One component of target validation is the use of genetically modified animal models. ln addition to drug development, the creation of loss of function ("knock-out") and gain of function ("knock-in") animal models are required for the basic elucidation of developmental pathways and disease states.

Human stem cells may be particularly useful in treating a wide range of diseases and conditions including autoimmune diseases, infectious diseases, organ and tissue transplant as well as traumatic injury and aging. Growing and maintaining human ESCs from a variety of sources provides a starting point for all subsequent applications. The task of growing human stem cells, however, is a daunting one. Current protocols for growing human stem cells, often involve the use of feeder cells (murine embryonic fibroblasts) grown in direct contact with the human ESCs or alternatively involve growing the human ESCs on a matrix, e.g. collagen, fibronectin or the like in the presence of media conditioned by murine embryonic fibroblasts.

ESCs are essential tools in the development of genetically modified mouse models. In order to ensure that the genetic alteration applied to the ESCs is maintained in the genotype of the engineered mice, the ESCs must retain their full pluripotence throughout culture manipulations, expansion and subsequent blastocyst injection. The requirement that the ESCs are maintained in the undifferentiated state is a general challenge during clone isolation and expansion. Current methods typically accomplish this by co-culturing ESCs with a mouse embryonic fibroblast (MEF) feeder layer in a co-culture where the two cell types are in direct contact. This process is currently performed in 96 well tissue culture treated plates.

The workflow is complicated involving culturing MEFs in the 96 well plate, mitotically inactivating the MEFs by gamma irradiation, or mitomycin C treatment, to prevent their overgrowth, and subsequently removing the MEFs before using the ESCs for blastocyst injection. In addition to the many steps involved, ESC-MEF co-cultures are very metabolically active requiring media changes twice a day due to the smaller media volumes in the 96 well format.

Thus conditions for growing and maintaining ESCs in vitro from any source are typically complex, time consuming, labor intensive, difficult and expensive. The need therefore exists for methods, systems and kits suitable for growing ESCs in vitro, which are relatively simple and easy to use, and which provide consistent results with a minimal expenditure of human and financial resources. Certain embodiments of the invention disclosed herein meet these needs.

### SUMMARY OF THE INVENTION

In certain embodiments, the invention relates to the surprising discovery that ESCs may be grown on the surface of a porous membrane. The porous membrane may be in fluid communication with a cell culture comprising a feeder cell, thereby eliminating the need for direct contact between the ESCs and either a feeder cell layer or a substrate comprising extra-cellular matrix proteins or both.

In some embodiments the invention provides a method of growing an ESC in vitro comprising a) contacting a surface of a porous membrane with an ESC; b) contacting the surface of a solid support with a feeder cell, e.g., an embryonic fibroblast, a fetal fibroblast; c) providing a culture media to the ESC; d) providing a culture media to the feeder cell, wherein the porous membrane is in fluid communication with the culture media of d).

In other embodiments the invention provides a method of growing an ESC in a tissue culture plate comprising one or more wells, where each of the wells comprises a porous membrane insert and a solid support positioned in fluid communication with the porous membrane insert, and where the method comprises a) contacting a surface of the porous membrane with an ESC; b) contacting a surface of the solid support, which lies beneath the porous membrane insert with a feeder cell; c) providing a culture media to the ESC; d) providing a culture media to the feeder cell.

In yet other embodiments the invention provides a system for growing ESCs in vitro comprising a solid support; a porous membrane in fluid communication with the solid support; and a culture media suitable for growing ESCs. The system may further comprise one or more of the following: feeder cells, (e.g., embryonic fibroblasts, fetal fibroblasts,) embryonic stem cells, media additives, mammalian serum, essential amino acids, antibiotics, an agent for genetically selecting transformants, growth factors, and leukemia inhibitory factor (LIF).

In still other embodiments the invention provides a system for growing ESCs comprising a tissue culture plate comprising one or more wells, where each of the wells comprises a porous membrane insert and a solid support positioned in fluid communication with the porous membrane insert, and a culture media suitable for growing ESCs. The system may further comprise feeder cells including fibroblasts, e.g., embryonic fibroblasts, fetal fibroblasts, and/or ESCs, media additives, mammalian serum, essential amino acids, antibiotics, an agent for genetically selecting transformants, growth factors, and leukemia inhibitory factor (LIF).

In further embodiments the invention provides a kit for growing ESCs comprising a solid support; a porous membrane in fluid communication with the solid support; at least one container; and instructions for growing ESCs. The kit may optionally comprise one or more of the following: culture media suitable for growing ESCs; culture media suitable for growing embryonic fibroblasts; ESCs; feeder cells, e.g., fetal fibroblasts, embryonic fibroblasts; one or more buffers or wash solutions, and media additives.

### BRIEF DESCRIPTION OF THE DRAWINGS

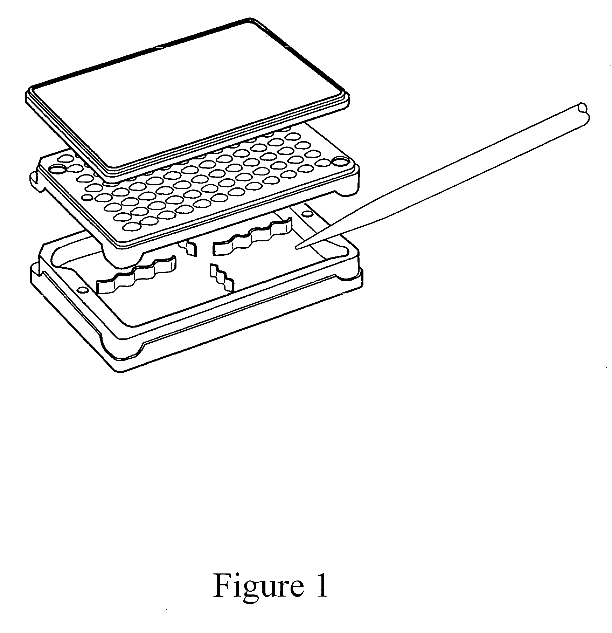

Figure 1 shows one example of a tissue culture plate suitable for use in certain embodiments of the invention comprising a multi-well plate suitable for growing ESCs in vitro.

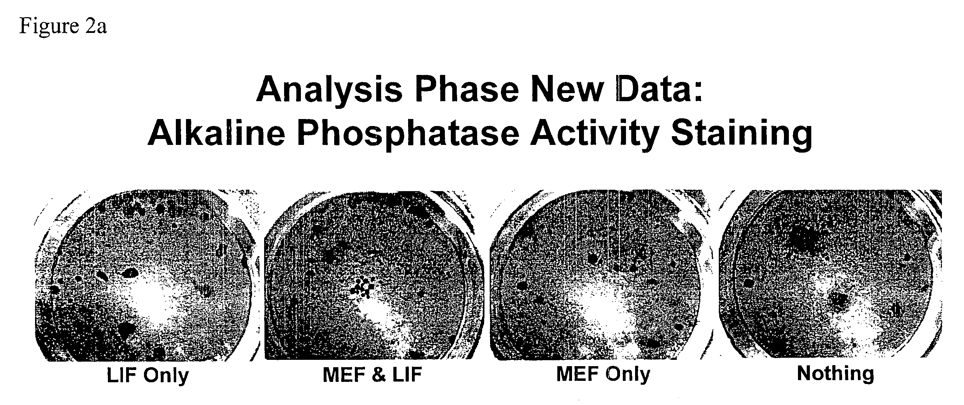

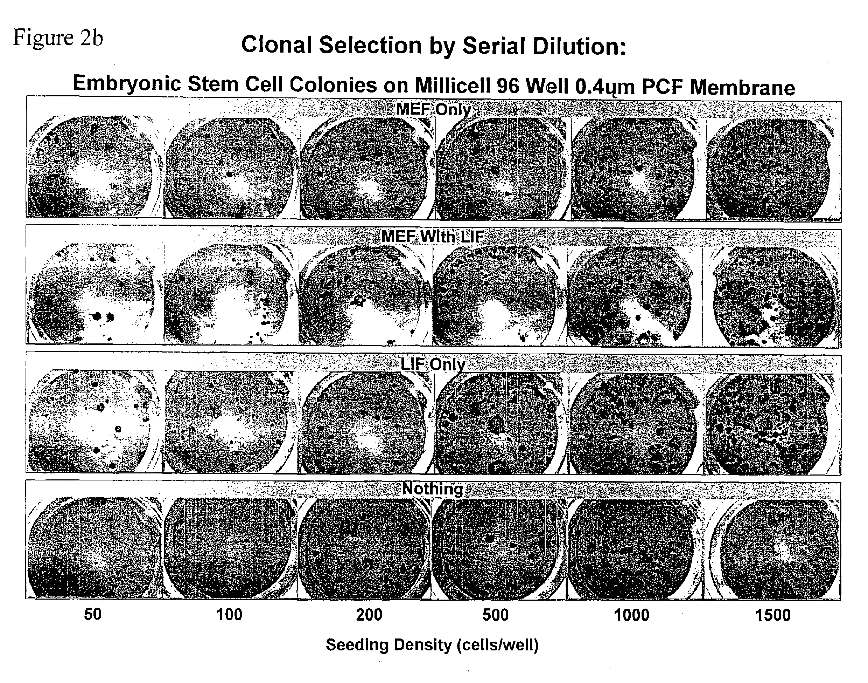

Figure 2 shows photographs of ESC cultures grown in vitro in 96 well plates where each well comprises a 0.4 µm PCF membrane upper well inserted into a plastic lower tray comprising feeder cells. Figure 2a shows ESC colonies grown under various conditions stained for alkaline phosphatase activity after 6 days. Figure 2b shows ESC colonies, grown under various conditions, serially diluted for clonal selection stained for alkaline phosphatase activity.

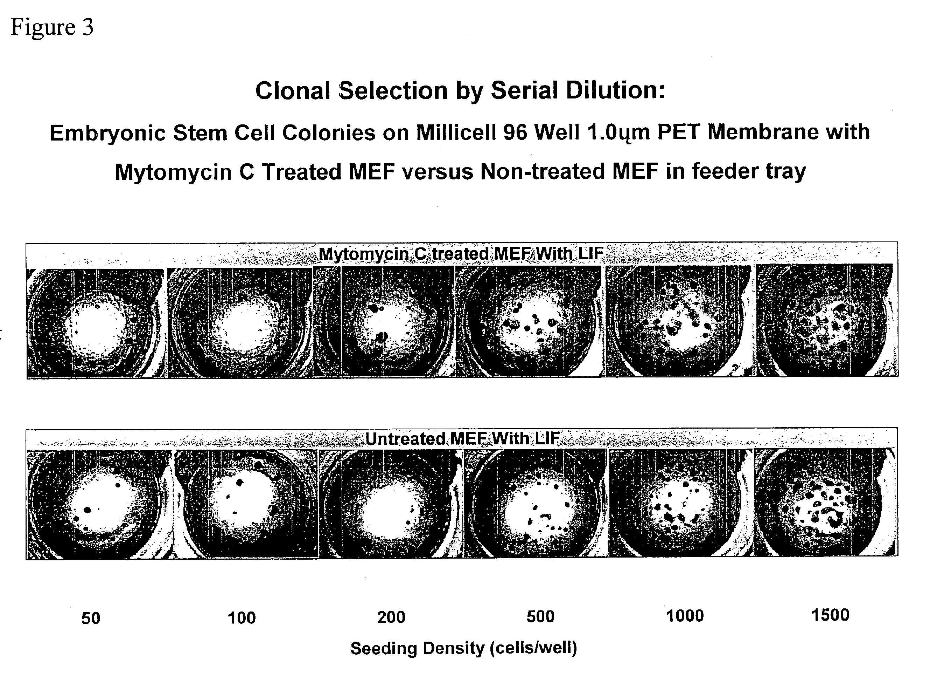

Figure 3 shows photographs of ESC cultures grown in vitro in 96 well plates where each well comprises a 1.0 µm PET membrane upper well inserted into a plastic lower feeder tray. The figures compare feeder cell layers treated with mytomycin C and leukemia inhibitory factor (LIF) or mytomycin C alone.

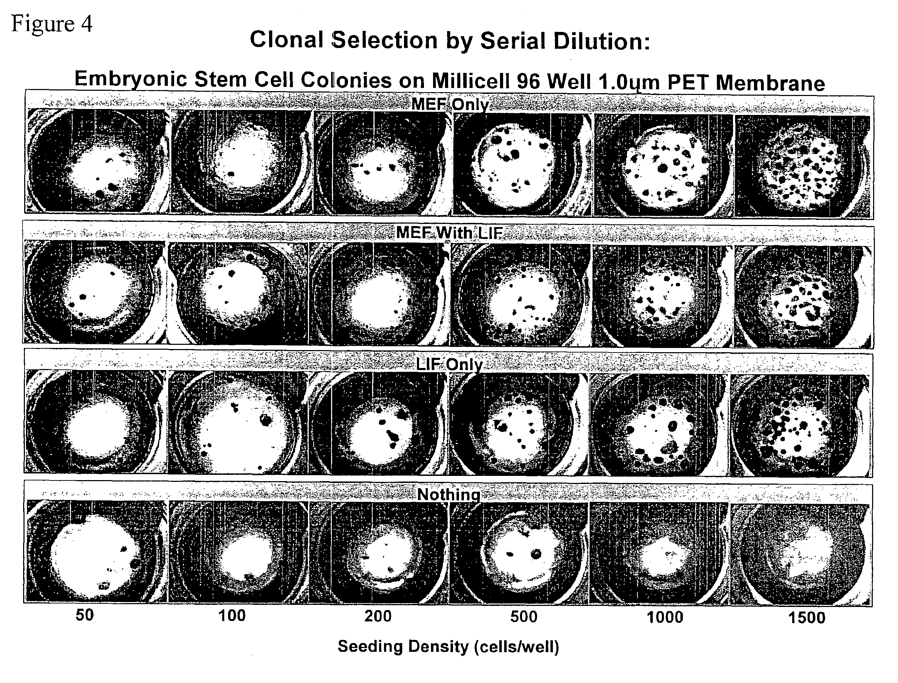

Figure 4 shows photographs of ESC cultures grown in vitro in 96 well plates where each well comprises a 1.0 µm PET membrane upper well inserted into a plastic lower feeder tray. The feeder well comprised either murine embryonic fibroblasts (MEF); MEF with LIF; LIF alone; or nothing in the feeder tray except for culture media.

### DESCRIPTION OF THE EMBODIMENTS

### Methods of Growing Embryonic Stem Cells

In certain embodiments the invention provides a method of growing ESCs in vitro that is simplified, reliable and relatively easy to execute. The cells may be maintained in an undifferentiated state. In some embodiments the method comprises co-culturing ESCs and a feeder cell but eliminates the need for direct physical contact between a feeder cell layer and an ESC layer. Thus the invention provides a method of growing ESC in vitro where the ESC may be physically and spatially separated from a feeder cell layer, e.g. by a porous membrane, such that media conditioned by the feeder cells is available for the ESCs. Several potential benefits result from physically separating the feeder cell layer from the ESC layer. Because the growth of the feeder cell layer no longer needs to be curtailed, the feeder cells no longer need to be mitotically inactivated.
Accordingly, the use of agents such as mitomycin C or gamma radiation may be eliminated. Both of these agents are potentially toxic and thus may raise concerns regarding, not only for the well being of the feeder cells themselves, but also, in the case of mitomycin C, regarding potential downstream uses of the ESCs generated under these conditions.

Physically separating the ESCs from the feeder cell layer also facilitates viewing the ESC colonies, e.g. by microscopy, and thus provides a means of better monitoring the growth and condition of the ESCs. Additionally the separation of the two layers facilitates the isolation and or harvesting of the ESC colonies for dilution, cloning or downstream manipulation, e.g. transfection, implantation into an animal or blastocyst.

In other embodiments the invention provides a method of growing an ESC on a porous membrane. The inventors have discovered that the surface of porous membranes are particularly well suited for growing ESC and thus eliminates the need for a substrate comprised of extracellular matrix proteins, typically used for growing human ESCs, thus saving both time and money by eliminating reagent expense.

The seeding density of the ESCs may be adjusted according to the size of the culture dish or well. The seeding density may be readily determined by the skilled artisan. Stem cells may be grown in order to expand clones from single cell isolates (e.g., by serial dilution) or for the expansion of established lines following cell splitting. In the former case, cells can be expanded by typical dilutions from a split culture to ensure cell density typical of stem cell expansion (i.e. 20-80% confluence) and then split again, e.g., 1:2; 1:4 depending on the desired density. In some embodiments, e.g., where the ESCs are of human origin, the cells may be grown until colonies reach a suitable size, irrespective of overall culture confluence, and then split. As an example, where the cells are to be seeded in a 96 well membrane plate (Figure 1) the ESCs may be seeded on a porous membrane at a number ranging from 1 to 3000 cells, 1-2000 cells, 1-1500 cells, 10-1000 cells, 20-500 cells, 50-300 cells per membrane.

Feeder cells may be seeded on a solid support, e.g., a plastic dish, or well positioned beneath a 96 well plate (Figure 1), at cell density ranging from 1-5000 cells, 1- 3000 cells, 1-2000 cells, 1-1500 cells, 10-1000 cells, 20-500 cells, 50-300 cells per well. Typically the feeder cells may be seeded such that the cells form a monolayer that results in an initial monolayer that is one third confluent. The cells may be allowed to grow to confluency before the ESCs are seeded on the membranes positioned in fluid communication with the feeder cell culture.

Suitable media for growing ESCs or feeder cells such as murine embryonic fibroblasts, include Dulbeco's Modified Eagle Media (Invitrogen, Carlsbad, CA). The skilled artisan will appreciate that a wide range of media suitable for growing stem cells in vitro are available, e.g., Specialty Media (Millipore Corporation, Billerica, MA); Resgro^{TM} (Millipore Corporation, Billerica, MA); StemXvivo (R&D Systems, Minneapolis, MN). The media may be supplemented with serum, e.g. fetal bovine serum, ES qualified serum (Invitrogen, Carlsbad, CA), antibiotics, e.g. Pen Strep (Invitrogen, Carlsbad, CA), non-essential amino acids (Invitrogen, Carlsbad, CA) and glutamine, e.g. Glutamax-1® (Invitrogen, Carlsbad, CA). Some ESC cultures may be further supplemented with leukemia inhibitory factor, e.g., Esgro® (Millipore Corporation, Billerida, MA).

### Cells

Some embodiments of the invention relates to co-culturing stem cells with a feeder cell line. Suitable stem cells may include any mammalian stem cell, e.g. stem cells originating from sources including a human, non-human primate, murine, rat, rabbit, bovine, goat, sheep, dog, cat, etc. In one embodiment the stem cells, e.g., ESCs may be murine ESCs. In another embodiment the stem cells may be, e.g., human ESCs.

The stem cells may be totipotent stem cells, which have the ability to form extra-embryonic membranes, tissues and the embryo itself. The stem cells may be pluripotent stem cells which have the ability to give rise to most tissues of an organism, e.g., tissues derived from endoderm, mesoderm or ectoderm. The stem cells may be multipotent stem cells such as hematopoietic stem cells.

The stem cells may be isolated from a cultured blastocyst and passaged one or more times in culture such that colonies, derived from a single progenitor may be selected. The stem cells may be genetically manipulated to express a desirable trait or genotype.

Standard techniques known in the art may be used to transfer genetic material into the cell, e.g. transfection using charged lipids or using electroporation. The transferred genetic material may include selectable markers such as hygromycin resistance or the like.

In certain embodiments it may be desirable to maintain the ESCs in an undifferentiated state. Several markers are available for monitoring the differentiation of ESCs. As an example high expression levels of cell surface proteins OCT-3/4 and SSEA-1 are indicative of undifferentiated ESCs. Similarly, high levels alkaline phosphatase activity are also indicative of undifferentiated ESCs. In contrast differentiation of ESCs is marked by the loss of markers such as Oct 3/4, alkaline phosphatase expression, SSEA (human or mouse specific), and/or the gain of germ layer specific markers.

Any suitable feeder cell may be used in the invention. A suitable feeder cell will allow growth and passage of the ESCs without significant cell death or differentiation.
Without being bound by any particular theory, it has been suggested by some that a feeder cell functions to keep ESCs alive and undifferentiated in culture by secreting appropriate cell factors, while others have more recently suggested that feeder cells function as a sponge and sequester growth factors produced by the ESCs. By sequestering factors which would normally result in ESC differentiation, the ESCs are maintained in an undifferentiated state. The feeder cells may be fibroblasts, e.g. embryonic fibroblasts, fetal fibroblasts. The fibroblasts may be derived from the umbilical cord. The fibroblasts may be mammalian in origin, e.g., human, murine etc.

### Solid Supports and Porous Membranes

Solid supports may be used in certain embodiments of the invention as a substrate for growing feeder cells. As an example of one embodiment of the invention, a multiwell plate may be employed for culturing ESCs. The plate may be comprised of a bottom tray comprised of a suitable material such as plastic which may be used for culturing feeder cells. The ESCs may then be cultured on inserts comprised of porous membranes which rest on top of the tray. The inserts may be comprised of an attachment or locking means to ensure that the inserts are stably attached to the bottom tray. Additionally, solid supports may be used as a base, for porous membranes, as described infra. Thus, the porous membranes may be seated on or partially postioned, e.g. around the edges, within a solid support. The solid support may be comprised of any plastic material including polymers such as polystyrene, polypropylene, polyethylene and thus may be configured as firm non-malleable surface which may be flat.

In some embodiments, the solid support may be comprised of a unitary design, e.g. a single tissue culture well, dish, plate, flask or the like. As an example the solid support may be configured as a single tissue culture dish comprised of a base having a single well, for growing feeder cells, and insert which rests on top of the base or within the well, wherein the insert is comprised of a solid support and a porous membrane suitable for growing ESCs. Alternatively, the solid support may be configured as a multiplex device. In this embodiment the base may be comprised of one or more wells suitable for growing feeder cells, while the insert may be configured as a multiwell plate comprising a solid support and a porous membrane suitable for growing ESCs. Typically, the solid support is configured such that the feeder cells are positioned basolaterally to the ESCs and the ESCs apical surface is exposed on the top of the porous membrane.

In some embodiments the solid support may be comprised of malleable polymers such as cellulose or cellulose acetate tubing used in hollow fiber bioreactor. If the tubing is porous it may serve both as a solid support and a porous membrane. As an example the ESCs may be grown on one side of the tubing and the feeder cells may be grown on the opposite side of the tubing. Alternatively both the feeder cells and the ESCs may be grown on separate tubing in the extra-capillary space around the tubing.

Porous membranes used in the invention may be comprised of any porous material known in the art. Examples of suitable porous materials, include, but are not limited to, polyethylene terephthalate, polymer cladded fibers (e.g. Millicell®)(Millipore Corp., Billerica, MA) polyether sulfone, polyamide, e.g., agarose, cellulose, a polysaccharide, polytetrafluoroethylene, polysulfone, polyester, polyvinylidene fluoride, polypropylene, a fluorocarbon, e.g. poly (tetrafluoroethylene-co-perfluoro(alkyl vinyl ether)), poly carbonate, polyethylene, glass, ceramic, nylon and metal.

### Systems

Certain embodiments of the invention provide for a system for growing stem cells in vitro. The system may comprise a solid support and a porous membrane in fluid communication with the solid support. The solid support may be suitable for growing feeder cells such as embryonic fibroblasts. The system may comprise one or more of the following: media suitable for growing ESCs; media suitable for growing feeder cells; feeder cells, wash buffers, and additional reagents for indirectly co-culturing ESC and feeder cells, e.g. LIF, fibronectin or other extracellular matrix proteins.

### Kits

The invention also provides, in some embodiments, for kits which may be used to grow ESC in vitro. The kit may comprise a solid support; a porous membrane in which may be suitably configured to be in fluid communication with the solid support; at least one container; and instructions for growing ESCs. Optionally, the kit may contain one more different cell culture media for growing ESCs and MEFs. The media may be provided in liquid or powder form. The kit may comprise cell culture reagents and supplements as well, e.g. glutamine, essential amino acids and the like. The supplements may be provided in liquid or powder form as well. Similarly, the kit may comprise one or more buffers and/or wash solutions. The buffers and wash solutions also may be provided in liquid or powder form. The kit may also comprise one more cell samples, e.g., ESCs, feeder cells such as MEFs. The cells may serve as starting materials for downstream applications or alternatively the cells may serve as control for future experiments. The cells may be provided frozen each in a separate container. The solid support and porous membrane may be provided in a pre-assembled form packaged and ready for use or in an unassembled form where the unassembled parts are optionally packaged separately. The instructions may be provided in one or more languages, e.g. English, French, German, Japanese etc.

### Examples

### Example 1: Fibronectin coating protocol

### Materials

Fibronectin 0.1 % Solution (Sigma, St Louis, MO)
Primary Mouse Embryo Fibroblasts strain CF-1 untreated (MEF)(Chemicon, Temecula, CA)
MEF Media:DMEM (Invitorgen, Carlsbad, CA)
10% Fetal Bovine Serum (Hyclone, Logan, UT)
1% Glutamax-1 (Invitorgen, Carlsbad, CA)
1% PenStrep (Invitorgen, Carlsbad, CA)
1% Non Essential Amino Acids (Sigma, St Louis, MO)
DPBS (Hyclone Logan, UT)

Fibronectin was diluted, (0.1 % Solution) (Sigma, St Louis, MO) to 25µg/mL in sterile DPBS. Enough Fibronectin in DPBS was added (25µg/mL) to coat a single well tray, (approximately 5-10mL per tray). The tray was incubated at room temperature for at least 45 minutes. Excess Fibronectin was removed before seeding MEF. MEF stored in a -80°C freezer were thawed. The MEF vial was gently shaken in a 37°C water bath. The MEF were transferred to a 15mL tube containing 10mL pre-warmed MEF media. The cells were pelleted at 1000 rpm for 4 minutes. The supernatant was removed and the cells resuspended in fresh pre-warmed MEF media.
A fibronectin coated single well feeder tray was seeded with MEF feeder cell suspension. 1.67X10⁶ MEF cells per single well tray were seeded and resulted in 95% confluence within 24 hours. The plate was covered with a lid and incubated at 37°C overnight.

### Example 2: Embryonic stem cell indirect co-culture with feeder cells

### Materials Used:

Millipore Cell Culture Filter Plates and Single Well Feeder Trays 24 well with 0.4µm PCF membrane or 1.0µm PET membrane.
96 well 0.4µm PCF membrane (Millipore Corp., Billerica, MA)
ESC Media: Knock out DMEM (Invitogen, Carlsbad, CA)
20% ES qualified Serum (Invitogen, Carlsbad, CA)
1% Glutamax-1 (Invitogen, Carlsbad, CA)
1% PenStrep (Invitogen, Carlsbad, CA)
1% Non Essential Amino Acids (Sigma, St Louis, MO)
0.1% ESGRO® (LIF) (Chemicon, Temecula, CA)
0.1 % 2-mercaptoethanol (Invitogen, Carlsbad, CA)

### MEF Media:

DMEM (Invitogen, Carlsbad, CA)
10% Fetal Bovine Serum (Hyclone, Logan UT)
1 % Glutamax-1 (Invitogen, Carlsbad, CA)
1% PenStrep (Invitogen, Carlsbad, CA)
1 % Non Essential Amino Acids (Sigma, St Louis MO)
Gelatin 2% Solution (Sigma, St Louis, MO)
Primary Mouse Embryo Fibroblasts strain CF-1 untreated (MEF) (Chemicon, Temecula, CA)
Mitomycin C powder (Sigma, St Louis, MO)
129/S6 Murine Embryonic Stem cells (ESC) (Chemicon, Temecula, CA)
DPBS (Hyclone, Logan, UT)
TrypLE™ Select (1X), liquid (Invitogen, Carlsbad, CA)
Alkaline phosphatase detection kit (Chemicon, Temecula, CA)
Fibronectin 0.1 % Solution (Sigma, St Louis, MO)

On day 1 a T75 flask was coated with 10mL 0.1 % Gelatin in DPBS and incubated for at least 30 minutes at 37°C. Mouse embryo fibroblasts (MEF) stored in a -80°C freezer were thawed and the MEF vial was gently shaken in a 37°C water bath. The MEF were transferred to 15mL tube containing 10mL pre-warmed MEF media. The cells were pelleted at 1000 rpm for 4 minutes. The supernatant was removed and the cells were resuspended in fresh pre-warmed MEF media. Excess gelatin was removed from the flask prior to seeding. The flask was seeded with the MEF feeder cell suspension. The cells were incubated at 37°C overnight. Using 3.75 - 5 X10⁶ MEF cells per 25mL MEF media in a T75 flask resulted in 95% confluence within 24 hours.
On day 2 the MEF feeder cells were treated with Mitomycin C to mitotically inactivate the cells. The Mitomycin C was prepared using a powdered stock that was 2mg. The powder was dissolved in 4mL of sterile H₂O to make a 500µg/mL solution. The working concentration used was 10µg/mL in MEF media. The cells were incubated at 37°C for 2 hours. The MEF media with Mitomycin C was removed and the cells were washed 3 times (10mL per wash for a T75 flask) with pre-warmed DPBS. New embryonic stem cell (ESC) media was added and the cells were incubated at 37°C until ready to seed ESC. Murine ESC stored in a liquid nitrogen tank were thawed. The vial containing the cells was gently shaken in a 37°C water bath. The ESC were transferred to a 15mL tube containing 10mL pre-warmed ESC media. The cells were pelleted at 1000 rpm for 4 minutes. The supernatant was removed and the cells were resuspended in fresh pre-warmed ESC media. A flask containing the mitotically inactivated MEF feeder layer and ESC media was seeded with the ESC suspension using 4.5X10⁶ ESC cells per 30mL ESC media in a T75 flask. The cells were incubated at 37°C overnight.
On day 3 the ESC on MEF feeder layer were fed with ESC media. On day 4 ESC on MEF feeder layer were fed with ESC media or passaged, at a 1:2 ratio, if required. Generally, the ESC are allowed to grow to a density ranging from 20-80% confluence and then split. The day 4 procedure was followed on days 5-8. After thawing ESC, 2-3 passages are typically performed before seeding onto a multi-well cell culture filter plate.
On day 9 the ESC on MEF feeder layer were fed with ESC media. A single well feeder tray was coated with fibronectin in DPBS and incubated for 45 minutes at room temperature. The stock solution was 0.1 % fibronectin (1000 µg/mL). The working concentration used was 25µg/mL in sterile DPBS. Enough fibronectin in DPBS (25µg/mL) was added to coat a single well tray, using approximately 5-10mL per tray. The excess fibronectin was removed, and the single well tray was ready for use.
MEF stored in a -80°C freezer were thawed. The cell vial was gently shaken in a 37°C water bath. The MEF were transferred to a 15mL tube containing 10mL pre-warmed MEF media. The cells were pelleted at 1000 rpm for 4 minutes.
The supernatant was removed and the cells were resuspended in fresh pre-warmed MEF media. A fibronectin coated single well feeder tray was seeded with a MEF feeder cell suspension. Using 1.67X10⁶ MEF cells per single well tray typically resulted in 95% confluence within 24 hours. The tray was covered with a lid and incubated at 37°C overnight.
On day 10 the ESC and MEF feeder cells from removed from the T75 flask according to the protocol that follows. The flask was washed 2X with pre-warmed DPBS (10mL each wash). The flask was allows to sit for 1-2 minutes per wash. The DPBS was removed and 3mL per flask of TrypLE (Invitrogen, Carlsbad, CA) was added. The flask was incubated at room temperature for 2-3 minutes. The flask was observed under a microscope, and when the cells were balled up and detached from flask ESC media was added to inactivate the TrypLE (Invitrogen, Carlsbad, CA). The flask was mixed well and washed to remove all cells from flask. The ESC were separated from MEF feeder cells as follows: the ESC/MEF cell suspension was transferred to a new T75 flask and incubated at 37°C for 45 minutes; the non-adherent cells were removed and transferred to a another new T75 flask and incubated at 37°C for 45 minutes; the non-adherent cells were removed and cell culture filter plate wells were seeded with the ESC suspension. 200 - 500 cells per well were seeded in 100µl ESC media to an apical well for a 96 well cell culture filter plate. 1000 -1500 cells per well were seeded in 400µl ESC media to an apical well for a 24 well cell culture filter plate. The MEF media from single well tray was removed and replaced with ESC media, using 32mL of ESC media per tray. The ESC seeded cell culture filter plates were added to the single well trays and the assembly was incubated at 37°C overnight.

On days 12 and 14 the ESC and MEF indirect co-culture was fed with ESC media. On day 16 the ESC were analyzed for alkaline phosphatase activity using a kit (Alkaline Phosphatate Detection Kit) (Serologicals Corporation, Norcross, GA), according to the manufacturer's instructions. The results demonstrated ESC grown on porous membranes having fluid communication with media from feeder cells remained undifferentiated (Figures 2 and 3). In experiments where LIF (ESGRO®)(Serologicals Corporation, Norcross, GA) was present in the media it was used at a concentration of 1000 units/ml throughout the expansion of test ESCs and experimentally at the same concentration (post initial expansion) where indicated.
All numbers expressing quantities of ingredients, reaction conditions, and so forth used in the specification and claims are to be understood as being modified in all instances by the term "about." Accordingly, unless indicated to the contrary, the numerical parameters set forth in the specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches. Many modifications and variations of this invention can be made without departing from its spirit and scope, as will be apparent to those skilled in the art. The specific embodiments described herein are offered by way of example only and are not meant to be limiting in any way. It is intended that the specification and examples be considered as exemplary only, with a true scope and spirit of the invention being indicated by the following claims.

## Claims

1. A method of growing an embryonic stem cells (ESC) in vitro comprising a) contacting a surface of a porous membrane with an ESC; b) contacting the surface of a solid support with a feeder cell; c) providing a culture media to the ESC; d) providing a culture media to the feeder cell, wherein the porous membrane is in fluid communication with the culture media of d).

2. The method of claim 1, wherein the ESCs are:
(a) maintained in an undifferentiated state; and/or
(b) chosen from murine embryonic stem cells and human embryonic stem cells.

3. The method of claim 1 or claim 2, wherein the feeder cell is:
(a) a fibroblast and/or
(b) a fibroblast chosen from a murine and a human fibroblast; and/or
(c) an embryonic fibroblast.

4. The method of claim 1, claim 2 or claim 3, wherein the ESC culture media comprises serum, and/or comprises LIF.

5. The method of any one of claims 1 to 4, wherein in the porous membrane and the solid support are provided in a multiwell plate; and optionally, wherein the multiwell plate is chosen from a plate comprised of 6, 12, 48 or 96 wells.

6. The method of any one of claims 1 to 5, wherein the porous membrane is comprised of a polymer; and optionally, wherein the polymer is chosen from polyethylene terephthalate, polymer cladded fibers, polyether sulfone, polyamide, agarose, cellulose, a polysaccharide, polytetrafluoroethylene, polysulfone, polyester, polyvinylidene fluoride, polypropylene, a fluorocarbon, e.g. poly (tetrafluoroethylene-coperfluoro(alkyl vinyl ether)), poly carbonate, polyethylene, glass, ceramic, nylon, metal and combinations thereof.

7. A system for growing ESCs in vitro comprising a solid support; a porous membrane in fluid communication with the solid support; and a culture media suitable for growing ESCs.

8. The system of claim 13 further comprising a feeder cell is:
(a) a fibroblast and/or
(b) a fibroblast chosen from a murine and a human fibroblast; and/or
(c) an embryonic fibroblast.

9. The system of claim 7 or claim 8, wherein in the porous membrane and the solid support are provided in a multiwell plate; and optionally, wherein the multiwell plate is chosen from a plate comprised of 6, 12, 48 or 96 wells.

10. The system of any one of claims 7 to 9, further comprising ESCs; and optionally, wherein the ESCs are chosen from murine ESCs and human ESCs.

11. A kit for growing ESCs comprising a solid support; a porous membrane in fluid communication with the solid support; at least one container; and instructions for growing ESCs.

12. The kit of claim 11, wherein in the porous membrane and the solid support are provided in a multiwell plate.

13. A method of growing an ESC in a tissue culture plate comprising one or more wells, where each of the wells comprises a porous membrane insert and a solid support positioned in fluid communication with the porous membrane insert, and where the method comprises a) contacting a surface of the porous membrane with an ESC; b) contacting a surface of the solid support, which lies beneath the porous membrane insert with an embryonic fibroblast; c) providing a culture media to the ESC; d) providing a culture media to the embryonic fibroblast.

14. The method of any one of claims 1 to 6 or 13, wherein the media is serum free.
